## Europäisches Patentamt
## European Patent Office
## Office européen des brevets

(19)

(11) Veröffentlichungsnummer: **0 066 272**
**B1**

(12) # EUROPÄISCHE PATENTSCHRIFT

(45) Veröffentlichungstag der Patentschrift:
**15.10.86**

(51) Int. Cl.⁴: **A 61 B 6/04**

(21) Anmeldenummer: **82104675.2**

(22) Anmeldetag: **27.05.82**

(54) **Patienten-Lagerungsvorrichtung mit einer drehbaren Lagerstatt.**

(30) Priorität: **01.06.81 DE 3121728**

(43) Veröffentlichungstag der Anmeldung:
**08.12.82 Patentblatt 82/49**

(45) Bekanntmachung des Hinweises auf die Patenterteilung:
**15.10.86 Patentblatt 86/42**

(84) Benannte Vertragsstaaten:
**DE FR SE**

(56) Entgegenhaltungen:
**DE-A-2 008 169**
**DE-A-2 343 862**
**DE-A-2 455 447**
**DE-C-968 128**
**FR-A-2 194 402**
**FR-A-2 291 733**
**FR-A-2 344 834**
**FR-A-2 353 269**
**FR-A-2 430 757**
**GB-A-2 075 810**

(73) Patentinhaber: **Siemens- Elema AB, Röntgenvägen 2, S-171 95 Solna 1 (SE)**
(84) Benannte Vertragsstaaten: **SE**

(73) Patentinhaber: **Siemens Aktiengesellschaft Berlin und München, Wittelsbacherplatz 2, D-8000 München 2 (DE)**
(84) Benannte Vertragsstaaten: **DE FR**

(72) Erfinder: **Larsson, Sten, Dipl.- Ing., Svärdsliljevägen 110, S-162 43 Vällingby (SE)**

LIBER, STOCKHOLM 1986

**Beschreibung**

Die Erfindung betrifft eine Patienten-Lagerungsvorrichtung mit einer um eine horizontale Raumachse drehbaren Lagerstatt für einen auf ihr ruhenden Patienten, die gegen ein eine Röntgenstrahlenquelle und eine Bilderfassungseinrichtung aufweisendes Röntgengerät höhen- (y-Koordinate), längs- (x-Koordinate) und seitenverstellbar ist, wobei zumindest die Höhenverstellung mittels eines Motors erfolgt, und mit einer Regelvorrichtung, die aus einer Anordnung für die Einstellung von Sollwerten für ein Isozentrum und einer weiteren Anordnung für die Ermittlung von Istwerten von Koordinaten der Lagerungsvorrichtung sowie einer Nachlaufsteuerung für den Ausgleich von Soll- und Istwerten besteht, wobei ein Drehmelder zur Bestimmung des Drehwinkels der Lagerstatt vorgesehen ist. Die Erfindung betrifft ferner ein Verfahren zur isozentrischen Verschiebung der Lagerstatt der Patienten-Lagerungsvorrichtung. Eine derartige Lagerungsvorrichtung ist beispielsweise aus der DE-AS 24 55 447 bekannt. Sie dient neuroradiologischen Untersuchungen, die eine Injektion von Kontrastmitteln in die Liquorräume erfordern, wie z.B. Encephalographie, Ventrikulographie und Myelographie. Insbesondere bei diesen Untersuchungen kommt es dabei darauf an, den schwerkraftabhängigen Kontrastmittelfluss auf einem Röntgenbildschirm zu verfolgen. Das Kontrastmittel wird in einen Rückenmarkskanal eingespritzt. Wird anschliessend der Patient mit dem Kopf nach unten geneigt, so rinnt das Kontrastmittel in Richtung auf den Kopf. Neigt man den Patienten anschliessend in die andere Richtung, so fliesst das Kontrastmittel wieder zurück.

Die bekannte Patienten-Lagerungsvorrichtung ermöglicht dabei eine isozentrische Drehung der Lagerstatt, um beispielsweise den Kontrastmittelfluss dirigieren zu können. Insbesondere bei Wirbelsäulenuntersuchungen ist es aber auch notwendig, den Kontrastmittelfluss bei variierender Neigung des Patienten über eine verhältnismässig lange Strecke zu verfolgen, die nur teilweise bei einer Durchleuchtung erfasst werden kann. Für eine einwandfreie Untersuchung ist es dabei wünschenswert, dass der Untersuchungspunkt ständig in der Mitte der Bilderfassungseinrichtung abgebildet wird, d.h. im Isozentrum.

Der vorliegenden Erfindung liegt daher die Aufgabe zugrunde, bei einer Patienten-Lagerungsvorrichtung der eingangs genannten Art bei einer durch eine Bedienungsperson vorgenommenen längswärtigen Verschiebung der Lagerungsvorrichtung automatisch eine derartige Höhenjustierung vorzunehmen, dass sich das Isozentrum auf einer im voraus festlegbaren Kurve entlang der Lagerstatt bewegt. Speziell soll sich das Isozentrum auf einer Parallelen zur Lagerstatt durch einen einmal festgesetzten Behandlungspunkt bewegen. Die Bewegung soll dabei unabhängig von der Winkellage der Lagerstatt und damit der Neigung des Patienten sein.

Diese Aufgabe wird erfindungsgemäss dadurch gelöst, dass die Patienten-Lagerungsvorrichtung 1 mit je einem Ist-Wert-Geber 10, 12 für eine x- bzw. y-Koordinate der horizontalen Raumachse (Drehachse der Lagerstatt) versehen ist, dass der Drehmelder 11 den Drehwinkel $\alpha$ der Lagerstatt 5 gegen ihre Horizontale bestimmt, und dass die Regeleinrichtung 22 aus diesen Werten und den Soll-Werten $x_a$, $y_a$ für das im Abstand d' zur Lagerstatt 5 befindliche Isozentrum ein Signal für die Nachlaufsteuerung 20 ermittelt, die den Motor 7 für die Höhenverstellung beim Auftreten einer Differenz zwischen dem Soll- und Ist-Wert des Isozentrums in y-Richtung $y_a$, $y_{is}$ im Sinn eines Ausgleichs betätigt (und wobei der Ist-Wert des Isozentrums z.B. als auf einer Parallelen im Abstand d' zur Lagerstatt liegend festgesetzt ist).

Die Patienten-Lagerungsvorrichtung hat dabei mindestens einen Motor für die Höhenverstellung der Lagerstatt. Die Längsverschiebung erfolgt manuell oder motorisch. Wenn ein Arzt die Lagerstatt um einen bestimmten Winkel gegen die Horizontale kippt, beginnt das Kontrastmittel zu fliessen. Um dabei das Kontrastmittel im Isozentrum zu behalten, nimmt der Arzt selbst eine Verstellung der Lagerstatt derart vor, dass die x-Koordinate des jeweiligen Untersuchungspunktes (Ort des Kontrastmittels) mit der x-Koordinate des Isozentrums übereinstimmt. Die Regeleinrichtung bestimmt dabei ständig für die aktuellen Koordinaten des Drehzentrums und den Kippwinkel $\alpha$ die Höhe des Untersuchungspunktes und vergleicht sie mit der Soll-Höhe des Isozentrums. Die Nachlaufsteuerung sorgt für einen ständigen Angleich dieser beiden Werte.

Mit Hilfe dieser Automatik ist es vorteilhaft möglich, unabhängig von der Neigung der Lagerstatt und unabhängig von der Verschiebung die Höhe selbständig so zu justieren, dass sich der Untersuchungspunkt stets im Isozentrum befindet. Auf diese Art und Weise kann das Fliessen des Kontrastmittels auf einfache und sichere Weise auf der Bilderfassungseinrichtung verfolgt werden.

Da es für viele Untersuchungen ausreicht, dass sich der Untersuchungspunkt in etwa im Isozentrum befindet, ist in Weiterbildung der Erfindung vorgesehen, dass der Ist-Wert des Isozentrums als auf einer Geraden im Abstand d' zur Lagerstatt festgesetzt ist. Beispielsweise bei einer Rückgratuntersuchung in Seitenlage eines Patienten folgt das Rückgrat annähernd einer solchen Geraden. Bei stärkeren Abweichungen besteht ausserdem die Möglichkeit, von Abschnitt zu Abschnitt manuell einen neuen Abstand d' einzustellen.

Die automatische Nachjustierung der Höhe der Patienten-Lagerungsvorrichtung stützt sich auf anhand der folgenden beispielhaft angeführte Überlegungen:

Zunächst sei eine Gerade parallel zur Lagerstatt- zumindest zu dem für die Untersuchung interessierenden Teil der Lagerstatt - durch die horizontale Raumachse angenommen. Diese Parallele ist durch die Koordinaten der Raumachse und den Kippwinkel festgelegt. Weiterhin ist angenommen, dass der Untersuchungspunkt sich entlang einer Parallelen zur Lagerstatt und damit zu der gedachten Linie durch die horizontale Raumachse bewegt. Der Abstand zwischen den beiden Parallelen ist jederzeit einstellbar. Zunächst wird dann der Abstand zwischen den beiden Parallelen ermittelt. Damit sind alle Werte vorgegeben, um für die bei der Untersuchung auftretenden und gemessenen Koordinatenwerte der horizontalen Raumachse die jeweilige Ist-Koordinate des Untersuchungspunktes in y-Richtung zu bestimmen und mit der Soll-Koordinate zu vergleichen.

In einer vorteilhaften Weiterbildung der Erfindung ist als Regeleinrichtung ein Microprozessor vorgesehen, der aus den eingegebenen Werten den Ist-Wert für die y-Koordinate des Isozentrums ermittelt und durch einen Vergleich mit dem entsprechenden Soll-Wert das Signal für eie Nachlaufsteuerung erzeugt, das dieser über einen Digital-Analogwandler zugeführt ist. Durch die Zurückführung der automatischen Höhenjustierung auf die bereits erwähnten mathematischen Beziehungen eignet sich der Microprozessor besonders gut für die Regelung. Die aktuellen Messwerte für die Lage der horizontalen Raumachse und für den Kippwinkel können analog abgegriffen und über Analog-Digitalwandler dem Microprozessor zugeführt werden.

Eine besonders vorteilhafte Ausführungsform sieht vor, dass mindestens für die x-Koordinate der horizontalen Raumachse ein digitalkodierter Positionsleser vorgesehen ist, dessen Signal unmittelbar dem Microprozessor zugeführt werden kann.

Eine besonders raumsparende und für den Arzt optimal zugängliche Patienten-Lagerungsvorrichtung ergibt sich in einer Weiterbildung der Erfindung durch Verwendung eines an sich bekannten verschiebbaren Deckenstatives mit einem in der Höhe verstellbaren Arm für die Lagerstatt. Diese Patienten-Lagerungsvorrichtung wird besonders vorteilhaft mit einem an einem C-Bogen-Stativ befestigten Röntgengerät kombiniert, das direkt Anordnungen für die Bestimmung der Soll-Werte des Isozentrums enthalten kann.

Im folgenden wird die Erfindung anhand eines in zwei Figuren beschriebenen Ausführungsbeispieles näher erläutert. Dabei zeigt

Fig. 1 in einer schematischen Darstellung die erfindungsgemässe Patienten-Lagerungsvorrichtung mit der automatischen Höhenjustierung und

Fig. 2 in perspektivischer Darstellung einen Ausschnitt aus der Patienten-Lagerungsvorrichtung in Verbindung mit einem Röntgengerät.

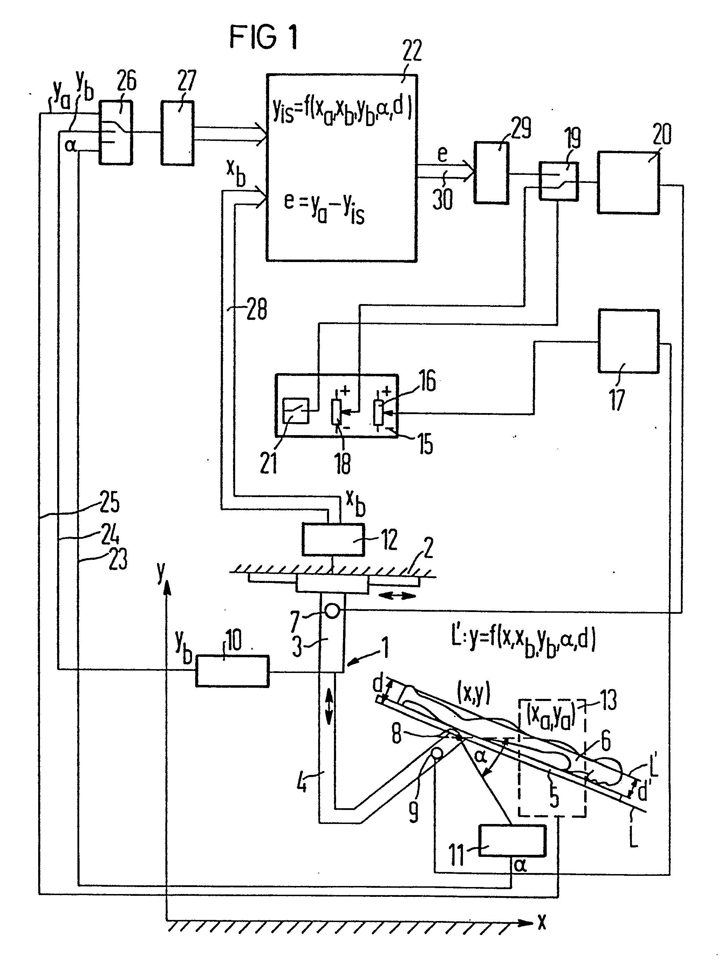

Die Patienten-Lagerungsvorrichtung 1 nach Fig. 1 besitzt eine an der Decke 2 verschiebbare Teleskopsäule 3, an deren ausfahrbarem Arm 4 eine Lagerstatt 5 für einen Patienten 6 drehbar gelagert ist. Die Lagerstatt 5 ist hier als durchgehendes Bett dargestellt. Es ist aber ebenso möglich, dass diese Lagerstatt in bekannter Weise aus Gelenkteilen aufgebaut ist. Die Höhenverstellung des Tragarmes 4 erfolgt mittels eines Motors 7. Die Verkippung der Lagerstatt um ihre Drehachse (horizontale Raumachse) 8 erfolgt hier ebenfalls mittels eines Motors 9.

Die Verschiebung entlang der Decke 2 erfolgt manuell. Mit der Teleskopsäule 3 ist ein Potentiometer 10 zur Bestimmung der y-Koordinate der Drehachse 8 verbunden. Weiterhin ist ein Drehmelder 11 an diesen Drehpunkt angeschlossen zur Bestimmung des Winkels $\alpha$.

In dem Ausführungsbeispiel gemäss der Fig. 1 ist zur Bestimmung der x-Koordinate der Drehachse 8 an der Decke ein digitalkodierter Positionsleser 12 vorgesehen.

Auf die Ausgestaltung des Röntgengerätes ist in dieser schematischen Darstellung nicht weiter eingegangen. Es ist lediglich als gestrichelter Kasten 13 dargestellt, dem die Soll-Werte für das Isozentrum entnommen werden können. An einem Steuerpult 15 kann mittels eines Potentiometers 16 über ein Steuerglied 17 der Motor 9 für die Verkippung der Lagerstatt 5 betätigt werden. Ebenso kann von diesem Steuerpult aus über ein Potentiometer 18 und einen Zweiwegeschalter 19 eine Nachlaufsteuerung 20 für den Motor 7 und damit für die Höhenverstellung der Lagerstatt betätigt werden. Ausserdem enthält das Steuerpult einen Schalter 21 zur Inbetriebnahme der Regelautomatik.

Als Regeleinrichtung dient im vorliegenden Ausführungsbeispiel ein Microcomputer 22. Die Messwerte für die Koordinaten des Drehzentrums sind mit $x_b$, $y_b$ und $\alpha$ bezeichnet. Die Messwerte für das Isozentrum lauten entsprechend $x_a$ und $y_a$. Abgesehen von der x-Koordinate des Drehzentrums liegen alle anderen Werte zunächst in analoger Form vor. Über Leitungen 23, 24 und 25 werden die Werte $y_a$, $y_b$ sowie $\alpha$ über einen Multiplexer 26 und einen Analog-Digitalwandler 27 dem Microprozessor 22 zugeführt. Die Zuführung des Wertes $x_a$ ist in der Figur nicht extra dargestellt. Der $x_b$-Wert des Drehzentrums wird direkt digital gemessen und kann über eine Leitung 28 direkt dem Microprozessor zugeführt werden. Der Microprozessor 22 ermittelt während des Automatikbetriebes ständig die Differenz e zwischen dem Ist- und dem Soll-Wert des Isozentrums ($e = y_a - y_{is}$). Dieser Wert wird über einen Digital-Analogwandler 29 und den Zweiwegeschalter 19 der Nachlaufsteuerung 20 zur Betätigung des Motors 7 zugeführt.

Die Funktionsweise der isozentrischen Verschiebung der erfindungsgemässen Patienten-Lagerungsvorrichtung ist nun folgende:

Zunächst wird bei ausgeschalteter Automatik der Ausgangspunkt der Untersuchung, z. B. der Injektionspunkt für das Kontrastmittel, in das Isozentrum gebracht. Anschliessend wird die Automatik eingeschaltet. Der Microprozessor 22 berechnet zunächst den Abstand d zwischen einer Parallelen L zur Lagerstatt 5 durch die Drehachse 8 und einer dazu Parallelen L' durch das Isozentrum. Danach berechnet der Microprozessor 22 laufend die y-Werte für die Gerade L' am Orte $x_a$ und bildet die Differenz e zwischen dem Soll-Wert $y_a$ und dem bestimmten Ist-Wert $y_{is}$. Dieser Differenzwert e bildet den Ausgangswert des Microprozessors 22 und wird über eine Leitung 30 dem Digital-Analogwandler 29 zugeführt. Das analoge Ausgangssignal des Digital-Analogwandlers 29 wird über den Zweiwegeschalter 19 der Nachlaufsteuerung 20 als Eingangssignal zugeführt. Ist der ermittelte Ist-Wert grösser als der Soll-Wert, so wird die Lagerstatt gesenkt. Ist der Ist-Wert kleiner als der Soll-Wert, wird die Lagerstatt angehoben. Dadurch wird unabhängig davon, wie die Lagerstatt 5 gekippt ist oder längswärts verschoben wird, die Höhe ständig so nachgeregelt, dass das Isozentrum entlang der Linie L' verschoben wird. Der Beobachtungspunkt liegt dadurch stets im Isozentrum.

Die Parallele zur Lagerstatt 5 durch die horizontale Drehachse 8 ist eine Funktion der Koordinaten dieses Drehzentrums $x_b$, $y_b$ und des Kippwinkels $\alpha$ und lässt sich schreiben als

$$- \sin \alpha \cdot x + \cos \alpha \cdot y - \cos \alpha \cdot y_b + \sin \alpha \cdot x_b = 0$$

Der Abstand zwischen dieser Geraden L und der dazu Parallelen L' durch das Isozentrum ist mit d bezeichnet und bildet eine Konstante, die sich in entsprechender Weise schreiben lässt als

$$d = - \sin \alpha_o \cdot x_a + \cos \alpha_o \cdot y_a - \cos \alpha_o \cdot y_{bo} + \sin \alpha_o \cdot x_{bo}$$

wobei $\alpha_o$, $x_{bo}$ und $y_{bo}$ die entsprechenden Werte zum Startpunkt der Automatik sind. Der Abstand zwischen der Lagerstatt 5 und der dazu Parallelen L' ist mit d' bezeichnet.

Entsprechend ergibt sich für die Gerade L' folgende Gleichung:

$$y = \frac{1}{\cos \alpha} (\sin \alpha \cdot x + \cos \alpha \cdot y_b - \sin \alpha \cdot x_b + d)$$

Für $x = x_a$ ergibt sich damit der Ist-Wert $y_{is}$ zu:

$$y_{is} = \frac{1}{\cos \alpha} (\sin \alpha \cdot x_a + \cos \alpha \cdot y_b - \sin \alpha \cdot x_b + d)$$

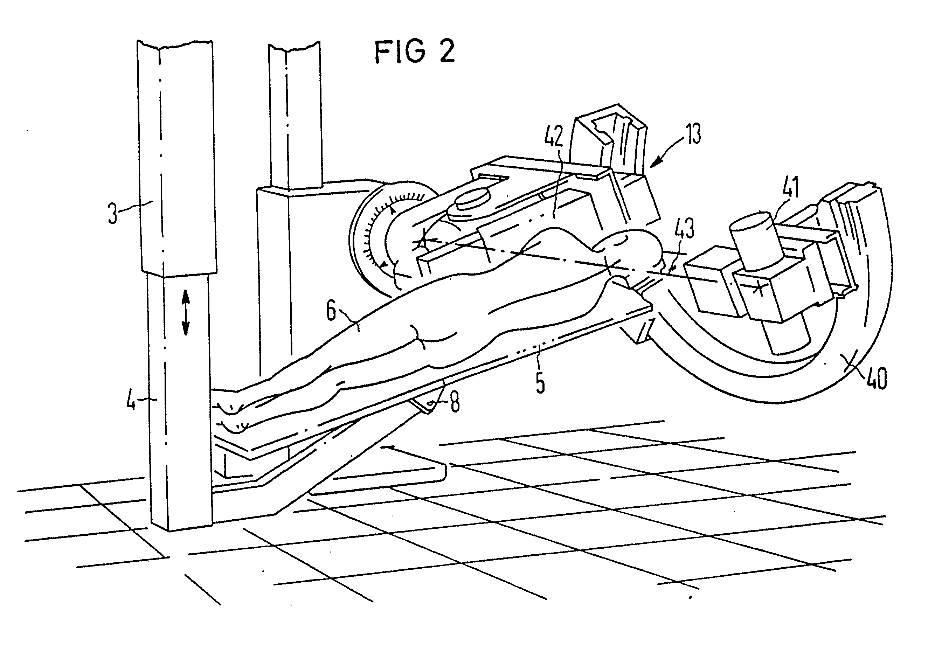

In Fig. 2 ist in perspektivischer Darstellung ein Ausschnitt der Patienten-Lagerungsvorrichtung 1 dargestellt. Gleiche Teile sind dabei mit gleichen Bezugszeichen versehen. Auf der Lagerstatt 5 ist ein in Seitenlage ruhender Patient 6 dargestellt. Die Lagerstatt ist drehbar an einem Tragarm 4 befestigt, der in vertikaler Richtung in einer Teleskopsäule 3 verschiebbar ist. Zusätzlich ist ein Röntgengerät 13 mit einem bekannten C-Bogen-Stativ 40 für eine Röntgenröhre 41 und einer Bilderfassungseinrichtung 42 dargestellt. Mit 43 ist der Zentralstrahl zwischen dem Fokus der Röntgenröhre und der Mitte des Bilderfassungsgerätes angedeutet, der durch das Isozentrum geht.

Wird beispielsweise im Nackenbereich Kontrastmittel in den Rückenmarkskanal eingespritzt, so fliesst bei der dargestellten Schräglage des Patienten das Kontrastmittel langsam in Richtung auf das Fussende des Patienten 6 den Rückenmarkskanal entlang.

Dieser Kanal liegt in einer im wesentlichen parallelen Ebene zur Lagerstatt 5. Mit Hilfe der beschriebenen Automatik zur Höhenjustierung ist es daher bei Längsverschiebung der Lagerstatt möglich, das Kontrastmittel stets im Isozentrum zu behalten.

**Patentansprüche**

1. Patienten-Lagerungsvorrichtung mit einer um eine horizontale Raumachse drehbaren Lagerstatt für einen auf ihr ruhenden Patienten, die gegen ein eine Röntgenstrahlenquelle und eine Bilderfassungseinrichtung aufweisendes Röntgengerät höhen- (y-Koordinate), längs- (x-Koordinate) und seitenverstellbar ist, wobei zumindest die Höhenverstellung mittels eines Motors erfolgt, und mit einer Regelvorrichtung, die aus einer Anordnung für die Einstellung von Sollwerten für ein Isozentrum und einer weiteren Anordnung für die Ermittlung von Istwerten von Koordinaten der Lagerungsvorrichtung sowie einer Nachlaufsteuerung für den Ausgleich von Soll- und Istwerten besteht, sowie einen Drehmelder zur Bestimmung des Drehwinkels der Lagerstatt aufweist, dadurch gekennzeichnet, dass die Patienten-Lagerungsvorrichtung (1) mit je einem Ist-Wert-Geber (10, 12) für eine x-bzw. y-Koordinate der horizontalen Raumachse (Drehachse der Lagerstatt) versehen ist, dass der Drehmelder (11) den Drehwinkel ($\alpha$) der Lagerstatt (5) gegen ihre Horizontale bestimmt, und dass die Regeleinrichtung (22) aus diesen Werten und den Soll-Werten ($x_a$, $y_a$) für das im Abstand d' zur Lagerstatt (5) befindliche Isozentrum ein Signal für die Nachlaufsteuerung

(20) ermittelt, die den Motor (7) für die Höhenverstellung beim Auftreten einer Differenz zwischen dem Soll- und Ist-Wert des Isozentrums in y-Richtung ($y_a$, $y_{is}$) im Sinn eines Ausgleichs betätigt.

2. Patienten-Lagerungsvorrichtung nach Anspruch 1, dadurch gekennzeichnet, dass der Soll-Wert des Isozentrums als auf einer Parallelen (L') im Abstand d' zur Lagerstatt (5) liegend festgesetzt ist.

3. Patienten-Lagerungsvorrichtung nach Anspruch 1 oder 2, dadurch gekennzeichnet, dass als Regeleinrichtung ein Microprozessor (22) vorgesehen ist, der aus den eingegebenen Werten den Ist-Wert für die y-Koordinate des Isozentrums ermittelt und durch einen Vergleich mit dem entsprechenden Soll-Wert das Signal für die Nachlaufsteuerung (20) erzeugt, das dieser über einen Digital-Analogwandler (29) zugeführt ist.

4. Patienten-Lagerungsvorrichtung nach Anspruch 3, dadurch gekennzeichnet, dass dem Microprozessor (22) eingangsseitig mindestens ein Analog-Digitalwandler (27) für die Umwandlung analoger Ist- oder Soll-Werte vorgeschaltet ist.

5. Patienten-Lagerungsvorrichtung nach einem der Ansprüche 1-4, gekennzeichnet durch ein in x-Richtung verschiebbares Deckenstativ (3) mit einem in der Höhe verstellbaren Arm (4) für die Lagerstatt (5).

6. Patienten-Lagerungsvorrichtung nach einem der Ansprüche 3-5, dadurch gekennzeichnet, dass mindestens für die x-Koordinate der horizontalen Raumachse (8) ein digitalkodierter Positionsleser (12) vorgesehen ist, dessen Signal unmittelbar dem Microprozessor (22) zugeführt ist.

7. Patienten-Lagerungsvorrichtung nach einem der Ansprüche 1-6, dadurch gekennzeichnet, dass für die Drehung der Lagerstatt (5) um die horizontale Raumachse (8) ein weiterer Motor (9) mit einer zugehörigen Steuerung (17) vorgesehen ist, über die die Drehung mittels eines Potentiometers (16) einstellbar ist.

8. Patienten-Lagerungsvorrichtung nach Anspruch 1 - 7, dadurch gekennzeichnet, dass das Röntgengerät (13) an einem separaten Stativ befestigt ist und Anordnungen für die Bestimmung der Soll-Werte des Isozentrums enthält.

9. Verfahren zur isozentrischen Verschiebung der Lagerstatt einer Patienten-Lagerungsvorrichtung nach einem der Ansprüche 1-8, dadurch gekennzeichnet, dass die Ist-Werte (x, y) der horizontalen Raumachse (8) (Drehachse der Lagerstatt) sowie der Drehwinkel ($\alpha$) der Lagerstatt (5) ermittelt werden, und dass bei einer Verschiebung der Lagerstatt (5) in x-Richtung durch die Regeleinrichtung (22) und die Nachlaufsteuerung (20) ständig die Höhe der Lagerstatt (5) derart justiert wird, dass sich das Isozentrum auf einer im voraus festlegbaren Kurve entlang der Lagerstatt bewegt.

10. Verfahren zur isozentrischen Verschiebung der Lagerstatt einer Patienten-Lagerungsvorrichtung nach Anspruch 9, dadurch gekennzeichnet, dass sich das Isozentrum entlang einer Parallelen (L') im Abstand (d') zur Lagerstatt (5) bewegt.

**Claims**

1. A patient supporting apparatus having a table which is rotatable about a horizontal spatial axis, for a patient resting thereon, which table is vertically (y-co-ordinate), longitudinally (x-co-ordinate) and laterally adjustable in relation to an X-ray apparatus which has an X-ray source and an image-detecting device, where at least the vertical adjustment is effected by means of a motor, having a control device which consists of an arrangement for adjusting theoretical values for an isocentre and a further arrangement for the determination of actual values of coordinates of the supporting apparatus, a follower control device for the balancing of theoretical and actual values, and a rotation indicator for determining the angle of rotation of the table, characterised in that the patient supporting apparatus (1) is provided with an actual value generator (10, 12) for an x- or y-co-ordinate of the horizontal spatial axis (axis of rotation of the table); that the rotation indicator (11) determines the angle of rotation ($\alpha$) of the table (5) relative to its horizontal; and that the control device (22) ascertains a signal for the follower control device (20) from said values and the theoretical values ($x_a$, $y_a$) for the isocentre which is located at the distance d' from the table (5), which follower control device operates the motor (7) for vertical adjustment in compensation when a difference between the theoretical and actual value of the isocentre in the y-direction ($y_a$, $y_b$) occurs.

2. A patient supporting apparatus as claimed in Claim 1, characterised in that the theoretical value of the isocentre is established as one that lies on a parallel (L') at the distance d' from the table (5).

3. A patient supporting device as claimed in Claim 1 or Claim 2, characterised in that as control device a microprocessor (22) is provided which determines the actual value for the y-co-ordinate of the isocentre from the input values and by a comparison with the corresponding theoretical value, produces the signal for the follower control device (20), which is supplied to the latter by means of a digital-analogue converter (29).

4. A patient supporting apparatus as claimed in Claim 3, characterised in that at the input end, the microprocessor (22) is proceeded by a least one analogue-digital converter (27) for the conversion of analogue actual or theoretical values.

5. A patient supporting apparatus as claimed in one of Claims 1 - 4, characterised by an overhead support (3) which is displaceable in the x-direction and has an arm (4) for the table (5), which is vertically adjustable.

6. A patient supporting apparatus as claimed in one of Claims 3 - 5, characterised in that a digital-coded position reader (12), whose signal is directly fed to the microprocessor (22), is provided at least for the x-co-ordinate of the horizontal spatial axis (8).

7. A patient supporting apparatus as claimed in one of Claims 1 - 6, characterised in that a further motor (9) is provided for the rotation of the table (5) about the horizontal spatial axis (8), which motor has a control unit (17) assigned to it, by means of which the rotation can be adjusted through a potentiometer (16).

8. A patient supporting apparatus as claimed in Claim 1 - 7, characterised in that the X-ray apparatus (13) is fixed on a separate support and comprises arrangements for the determination of the theoretical values of the isocentre.

9. A process for the isocentric displacement of the table of a patient supporting apparatus as claimed in one of Claims 1 - 8, characterised in that the actual values (x, y) of the horizontal spatial axis (8) (axis of rotation of the table) and the angle of rotation (α) of the table (5) are determined; and that, during a displacement of the table (5) in the x-direction, the height of the table (5) is constantly adjusted by the control device (22) and the follower control device (20) in such manner that the isocentre moves along the table on a curve which can be defined in advance.

10. A process for the isocentric displacement of the table of a patient supporting apparatus as claimed in Claim 9, characterised in that the isocentre moves along a parallel (L') at the distance (d') from the table (5).

**Revendications**

1. Dispositif formant couchette pour patient, comportant une couchette pouvant pivoter autour d'un axe horizontal et prévue pour un patient qui s'y trouve allongé, et qui est réglable en hauteur (coordonnées y), longitudinalement (coordonnées x) et latéralement par rapport à un appareil radiologique comportant une source de rayons X et un dispositif d'enregistrement d'images, au moins le réglage en hauteur s'effectuant à l'aide d'un moteur, et comportant un dispositif de réglage qui est constitué par un dispositif servant à régler des valeurs de consigne pour un isocentre et un autre dispositif servant à déterminer les valeurs réelles de coordonnées du dispositif formant couchette ainsi qu'un système de servocommande servant à égaliser des valeurs de consigne ou des valeurs réelles, ainsi qu'un synchro-transmetteur servant à déterminer l'angle de rotation de la couchette, caractérisé par le fait que le dispositif formant couchette pour patient (1) comporte des générateurs respectifs de valeurs réelles (10, 12) pour des coordonnées x ou y de l'axe horizontal (axe de rotation de la couchette), que le synchro-transmetteur (11) détermine l'angle de rotation (α) de la couchette (5) par rapport à sa position horizontale, et que le dispositif de réglage (22) détermine, à partir de ces valeurs et des valeurs de consigne (x$_a$,y$_a$) pour l'isocentre situé à la distance d' de la couchette (5), un signal pour le système de servocommande (20) qui actionne le moteur (7) pour le réglage en hauteur, dans le sens d'une compensation, lors de l'apparition d'une différence entre la valeur de consigne et la valeur réelle de l'isocentre suivant la direction y (y$_a$, y$_{is}$).

2. Dispositif formant couchette pour patient suivant la revendication 1, caractérisé par le fait que la valeur de consigne de l'isocentre est fixée comme étant située sur une parallèle (L') à la couchette (5), à une distance d' de cette dernière.

3. Dispositif formant couchette pour patient suivant la revendication 1 ou 2, caractérisé par le fait qu'il est prévu, comme dispositif de réglage, un microprocesseur (22) qui détermine la valeur réelle de la coordonnée y de l'isocentre à partir des valeurs introduites et produit, au moyen d'une comparaison avec la valeur de consigne correspondante, le signal destiné au système de servocommande (20) et qui est envoyé à ce dernier par l'intermédiaire d'un convertisseur numérique/analogique (29).

4. Dispositif formant couchette pour patient suivant la revendication 3, caractérisé par le fait qu'au moins un convertisseur analogique/numérique (27) servant à réaliser la conversion de valeurs réelles ou de valeurs de consigne analogiques est branché, côté entrée, en amont du microprocesseur (22).

5. Dispositif formant couchette pour patient, suivant l'une des revendications 1-4, caractérisé par un statif plafonnier (3) déplaçable suivant 12 direction x et comportant un bras (4); réglable en hauteur, de support de 12 couchette pour patient (5).

6. Dispositif formant couchette pour patient suivant l'une des revendications 3-5, caractérisé par le fait qu'au moins pour la coordonnée x de l'axe horizontal (8), il est prévu un lecteur de position (12) codé sous forme numérique et dont le signal est envoyé directement au microprocesseur (22).

7. Dispositif formant couchette pour patient suivant l'une des revendications 1-6, caractérisé par le fait que pour la rotation de la couchette (5) autour de l'axe horizontal (8) il est prévu un moteur supplémentaire (9) comportant un dispositif de commande associé (17), au moyen duquel la rotation peut être réglée à l'aide d'un potentiomètre (16).

8. Dispositif formant couchette pour patient suivant les revendications 1-7, caractérisé par le fait que l'appareil radiologique (13) est fixé sur un statif séparé et contient des dispositifs pour la détermination des valeurs de consigne de l'isocentre.

9. Procédé pour déplacer de façon isocentrique la couchette d'un dispositif à couchette pour patient suivant l'une des revendications 1-8, caractérisé par le fait qu'on détermine les valeurs réelles (x,y) de l'axe horizontal (8) (axe de rotation de la couchette) ainsi que l'angle de rotation ($\alpha$) de la couchette (5) et que dans le cas d'un déplacement de la couchette (5) suivant la direction x, la hauteur de la couchette (5) est réglée en permanence par le dispositif de réglage (22) et par le système de servocommande (20) de manière que l'isocentre se déplace sur une courbe fixée par avance, le long de la couchette.

10. Procédé pour réaliser le déplacement isocentrique de la couchette d'un dispositif à couchette pour patient suivant la revendication 9, caractérisé par le fait que l'isocentre se déplace le long d'une parallèle (L') à la couchette (5), à une distance (d') de cette dernière.

# FIG 1

$$y_{is}=f(x_a,x_b,y_b,\alpha,d)$$

$$e=y_a-y_{is}$$

$$L':y=f(x,x_b,y_b,\alpha,d)$$

FIG 2